# EUROPEAN PATENT SPECIFICATION

(11) **EP 1 847 230 B1**
(45) Date of publication and mention of the grant of the patent: **27.10.2010**
(21) Application number: 07007783.9
(22) Date of filing: 17.04.2007
(51) Int. Cl.: A61B 18/12, A61B 18/16, A61B 17/00

(54) **System for reducing patient return electrode current concentrations**
System zur Verringerung der Neutralelektrodenstromkonzentrationen
Système de réduction des concentrations de courant d'une électrode de retour d'un patient

(30) Priority: 18.04.2006 US 406012
(43) Date of publication of application: 24.10.2007
(73) Proprietor: Covidien AG, 8212 Neuhausen am Rheinfall (CH)
(72) Inventor: Bahney, Timothy J., Portland, Oregon 97209 (US)
(74) Representative: HOFFMANN EITLE

(56) References cited:
- WO-A-00/65993
- CA-A1- 1 219 642
- US-A1- 2005 101 947
- US-A1- 2006 074 411

## Description

### BACKGROUND

### Technical Field

The present invention is directed to a return electrode for use in an electrosurgical system, and to an electrosurgical system comprising the same. The invention may find application in patient return electrode systems and methods for performing monopolar surgery and RF ablation using the same.

### Background

During electrosurgery, a source or active electrode delivers energy, such as radio frequency energy, from an electrosurgical generator to a patient. A return electrode carries the current back to the electrosurgical generator. In monopolar electrosurgery, the source electrode is typically a hand-held instrument placed by the surgeon at the surgical site and the high current density flow at this electrode creates the desired surgical effect of cutting, ablating and/or coagulating tissue. The patient return electrode is placed at a remote site from the source electrode and is typically in the form of a pad adhesively adhered to the patient.

The return electrode typically has a relatively large patient contact surface area to minimize heat concentrations at that patient pad site (i.e., the smaller the surface area, the greater the current density and the greater the intensity of the heat.) Hence, the overall area of the return electrode that is adhered to the patient is generally important because it minimizes the chances of current concentrating in any one spot which may cause patient burns. A larger surface contact area is desirable to reduce heat intensity. The size of return electrodes is based on assumptions of the anticipated maximum current during a particular surgical procedure and the duty cycle (i.e., the percentage of time the generator is on) during the procedure. The first types of return electrodes were in the form of large metal plates covered with conductive jelly. Later, adhesive electrodes were developed with a single metal foil covered with conductive jelly or conductive adhesive. However, one problem with these adhesive electrodes was that if a portion thereof peeled away from the patient, the contact area of the electrode with the patient decreased, thereby increasing the current density at the adhered portion and, in turn, increasing the heat applied to the tissue. This resulted in an increased risk of burning the patient in the area under the adhered portion of the return electrode if the tissue was heated beyond the point where normal circulation of blood could cool the skin.

To address this problem, split return electrodes and hardware circuits, generically called Return Electrode Contact Quality Monitors (RECQMs), were developed. These split electrodes consist of two separate conductive foils arranged as two halves of a single return electrode. The hardware circuit uses an AC signal between the two electrode halves to measure the impedance therebetween. This impedance measurement is indicative of how well the return electrode is adhered to the patient since the impedance between the two halves is directly related to the area of patient contact. That is, if the electrode begins to peel from the patient, the impedance increases since the contact area of the electrode decreases. Current RECQMs are designed to sense this change in impedance so that when the percentage increase in impedance exceeds a predetermined value or the measured impedance exceeds a threshold level, the electrosurgical generator is shut down to reduce the chances of burning the patient.

As new surgical and therapeutic RF procedures continue to be developed that utilize higher current and higher duty cycles, increased heating of tissue under the return electrode may occur. Ideally, each conductive pad would receive substantially the same amount of current, therefore reducing the possibility of a pad site burn. However, this is not always possible due to patient size, incorrect placement of pads, differing tissue consistencies, etc.

US 2005/0101947 A1 discloses apparatus for dispersing return current flow in monopolar electrosurgery.

CA 1 219 642 A1 discloses a combination of features falling within the scope of the preamble of Claim 1.

### SUMMARY OF THE INVENTION

According to the present invention, there is provided a return electrode for use in an electrosurgical system, the return electrode comprising: a non-conductive pad having a top surface and a bottom surface; and a plurality of concentric, electrically isolated conductive elements coupled to the top surface of the non-conductive pad, concentrically outer conductive elements surrounding concentrically inner conductive elements, wherein each conductive element defines a leading edge to be located in relatively close proximity to a source of electrosurgical energy in an electrosurgical system, wherein each conductive element is independently electrically connectable to the source of electrosurgical energy, characterized in that: a current detection system is connected to each conductive element between the top surface and the bottom surface of the non-conductive pad.

With embodiments designed according to the present invention, a return electrode pad can be provided which has the ability to relieve large current concentrations on a leading edge thereof and to make adjustments so as to reduce the current concentration at said leading edge thereof, thereby reducing the likelihood of patient burns.

### BRIEF DESCRIPTION OF THE DRAWINGS

To enable a better understanding of the present invention, and to show how the same may be carried into effect, reference will now be made, by way of example only, to the accompanying drawings, in which:
FIG. 1 is a schematic illustration of a monopolar electrosurgical system;
FIG. 2 is a plan view of one embodiment of an electrosurgical return electrode according to the present invention, illustrating a conductive pad having a plurality of concentric conductive elements;
FIG. 3 is a cross-sectional view of the electrosurgical return electrode as taken through 3-3 of FIG. 2;
FIG. 4 is an enlarged schematic cross-sectional view of a portion of the return electrode of FIGS. 2 and 3; and
FIG. 5 is an electrical schematic of the electrosurgical system of FIG. 1 employing the return electrode of FIGS. 2-4.

### DETAILED DESCRIPTION

The following description is directed to patient return electrodes and electrosurgical systems, and methods for performing monopolar surgery and RF ablation using the same.

Exemplary embodiments of the electrosurgical system and methods of using the same are described herein with reference to the accompanying drawing figures, in which like reference numerals identify similar or identical elements. In the following description, well-known functions or constructions are not described in detail to avoid obscuring the disclosure in unnecessary detail.

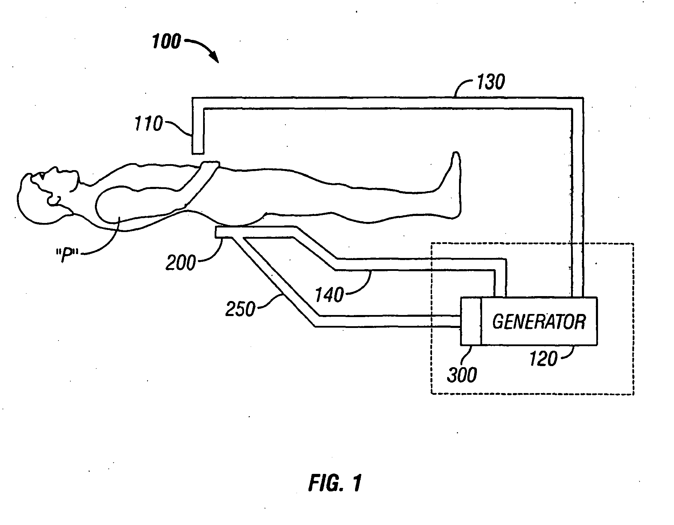

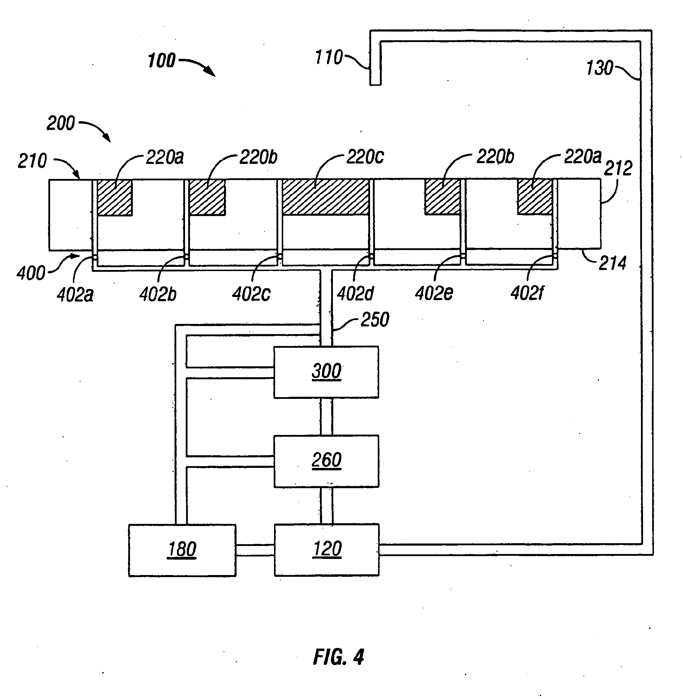

Referring initially to FIG. 1, a schematic illustration of a monopolar electrosurgical system 100 is shown. The electrosurgical system 100 includes a surgical instrument 110 (e.g., electrosurgical pencil, electrical scalpel, or other suitable active electrode) selectively connectable or permanently connected to a generator 120, a return electrode 200, a connection device 300 for connecting the return electrode 200 to generator 120, and a current detection system 400 disposed on or associated with the return electrode 200 (see FIG. 4). In FIG. 1, return electrode 200 is illustrated in an operative position placed beneath a patient "P." Electrosurgical energy is supplied to the surgical instrument 110 by the generator 120 via a cable 130 to cut, coagulate, blend, etc. tissue. The return electrode 200 returns energy delivered by the surgical instrument 110 to the patient "P" back to the generator 120 via return path 140. In certain instances, the electrosurgical energy flows from return electrode 200, through patient "P", to surgical instrument 110, and on to generator 120.

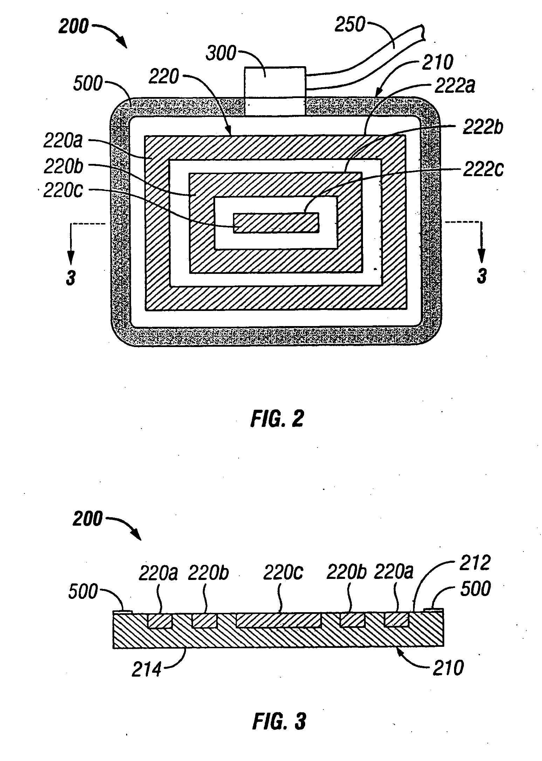

As seen in FIGS. 1 and 4, a current detection system 400 is associated with return electrode 200 and is coupled to connection device 300 via a cable 250. Connection device 300 may be coupled to generator 120 (FIG. 1), may be coupled to return electrode 200 (FIGS. 2 and 3), may be disposed between return electrode 200 and a generator 120 (FIG. 4), or may be housed within generator 120.

Turning now to FIGS. 2 and 3, a detailed discussion of one embodiment of return electrode 200, for use in monopolar surgery, follows. Return electrode 200 includes a non-conductive pad 210 having a top surface 212 and a bottom surface 214. Return electrode 200 is designed and configured to receive current during monopolar electrosurgery. While the figures depict return electrode 200 in a generally rectangular shape, return electrode 200 may have any suitable regular or irregular geometric shape, including and not limited to triangular, rectangular, circular, polygonal, etc.

Return electrode 200 includes a plurality of conductive elements 220 supported on top surface 212 of pad 210. As seen in FIG. 2, return electrode 200 includes a plurality of concentric conductive elements 220a-220c supported on or disposed within top surface 212 of pad 210. Conductive elements 220a-220c are electrically isolated from one another.

In the illustrated embodiment a first conductive element 220a surrounds a second conductive element 220b, while second conductive element 220b surrounds a third conductive element 220c. Each conductive element 220a-220c may be formed as a foil of conductive material or other suitable highly conductive media.

While three conductive elements 220 are shown and described, return electrode 200 may include any suitable number of conductive elements 220. Additionally, each conductive element 220 may have a uniform width or may have a varying width along the length thereof. Also, each conductive element 220 may have a different width as compared to other conductive elements 220 of return electrode 200. Any combination of these arrangements and/or configurations of conductive elements 220 falls within the scope of the present disclosure.

In an embodiment the number of conductive elements 220 and the area/size/dimension of each conductive element 220 is selected and/or optimized in order to achieve the greatest effect in dissipating current concentrations and/or temperature concentrations.

Each conductive element 220a-220c defines a leading edge 222a-222c, respectively. As used herein, the term "leading edge" is defined as the edge or corner of each conductive element 220a-220c that is closest to the source of energy or generator 120, or closest to connection device 300.

Each conductive element 220a-220c is separately and independently electrically connected or connectable to generator 120 and/or to a processor or computer 180, as seen in FIG. 4. In operation, computer 180 switches conductive elements 220a-220c into and out of the electrosurgical circuit (i.e., between being capable of transmitting/receiving electrosurgical current/energy and being incapable of transmitting/receiving electrosurgical current/energy) in order to move the leading edges 222a-222c to different locations along return electrode 200. In so doing, any current concentrations and/or temperature concentrations that may occur are distributed along the surface of return electrode 200.

In operation, according to one method, computer 180 may switch the conductive elements 220a-220c into or out of the circuit at predefined or predetermined intervals, may sequentially cycle through conductive elements 220a-220c according to said predefined or predetermined intervals and/or may randomly cycle through conductive elements 220a-220c according to said predefined or predetermined intervals.
According to another method of operation, computer 180 may switch the conductive elements 220a-220c into or out of the circuit in response to inputs received and/or temperature measurements taken at the patient/return-electrode interface and/or temperature measurements taken within return electrode 200.
As illustrated in FIG. 4, current detection system 400 includes an array of individual current sensors (illustrated as 402a-402f, distributed among conductive elements 220a-220c), which current sensors are able to measure the amount of current returning to conductive elements 220a-220c. The current detection system 400 may be coupled to the plurality of conductive elements 220a-220c anywhere between top surface 212 and bottom surface 214 of non-conductive pad 210. For example, individual current sensors 402a, 402f may be coupled to conductive element 220a, individual current sensors 402b, 402e may be coupled to conductive element 220b, and individual current sensors 402c, 402d may be coupled to conductive element 220c.
In an embodiment, the leading edge 222a-222c of each respective conductive element 220a-220c may include at least one discrete current sensor 402 operatively connected thereto. The current sensors operatively associated with the leading edges 222a-222c of conductive elements 220a-220c are independent of the current sensors operatively associated with the remainder of conductive elements 220a-220c. Moreover, each current sensor 402a-402f may be connected via a common cable 250 to a comparator 180 disposed within connection device 300 or generator 120.

Generally, the area of the return electrode 200 that is in contact with the patient "P" affects the current density of a signal that heats the patient "P." The greatest current density usually occurs along the leading edge of the conductive elements of the return electrode. Typically, the smaller the contact area of return electrode 200 with the skin of the patient "P," the greater the current density and in turn the greater the heating of tissue at the contact site. Conversely, the greater the contact area of return electrode 200 with the skin of the patient "P," the smaller the current density and in turn the smaller the heating of tissue at the contact site.

As can be appreciated and as mentioned above, higher current densities may be located along the leading edges of the conductive elements of the return electrodes, which lead to greater heating of tissue and greater probability of patient burn in the areas where the leading edges of the conductive elements of the return electrodes are in contact with the skin of the patient "P". It is therefore important to either ensure a relatively high amount of contact area between return electrode 200 and the patient "P," or otherwise maintain a relatively low current density on the return electrode 200.

While there are various methods of maintaining a relatively low current density the present disclosure ensures relatively low current densities along the leading edges 222a-222c of conductive elements 220a-220c. This may be accomplished by sensing the amount of current returning to each of the plurality of conductive elements 220a-220c of the return electrode 200 and switching the conductive elements 220a-220c out of the circuit in response to inputs received and/or temperature measurements taken at the patient/return-electrode interface and/or temperature measurements taken within return electrode 200, thereby reducing current densities at the patient site.

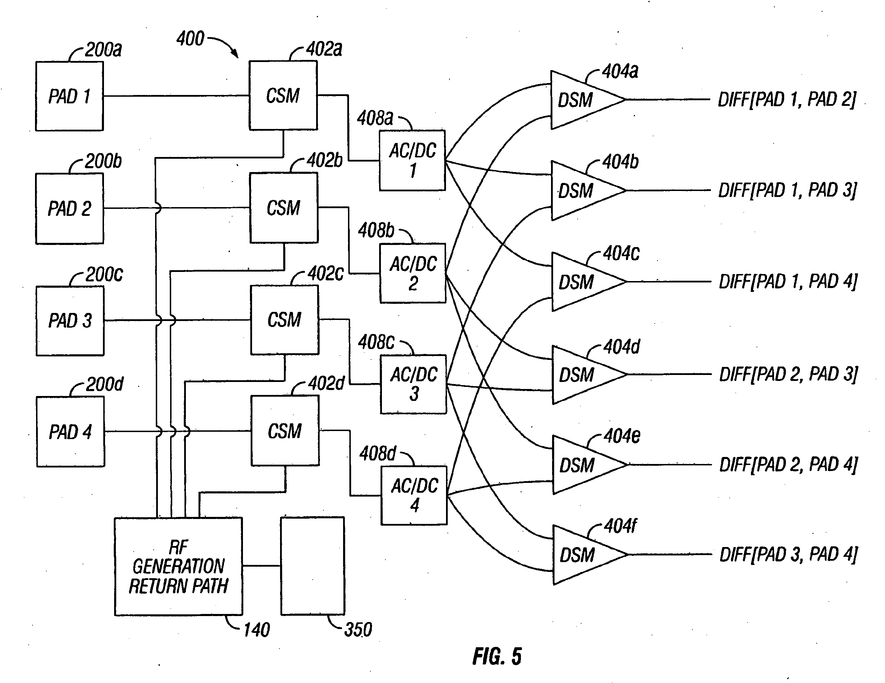

Referring now to FIG. 5, current detection system 400 may be associated with a plurality of return electrodes 200a-200d that are each coupled to generator 140. One or more algorithms control(s) the electrical energy associated with each return electrode 200a-200d to reduce patient burn. As seen in FIG. 5, current detection system 400 may include a sensing device 402a for sensing the current to each return electrode 200a-200d. Current detection system 400 may further include a plurality of comparators 404a-404f that sense the difference in current for respective return electrodes 200a-200d. Current detection system 400 is connected to each return electrode 200a-200c and may be located in a variety of different areas including, on the conductive elements thereof, inside connection device 300, or within generator 120. Other locations for current detection system 400 are within the scope of the present disclosure.

Current sensor(s) 402a-402d may take a number of suitable forms including, but not limited to, open loop sensors, closed loop sensors, digital current sensors, Hall-effect devices or a current sense transformer (not shown), the operation of which is described hereinbelow. In use, the return current for each return electrode 200a-200d is passed through a toroidal magnetic, which forms a 1:N current sense transformer comprised of 1 turn from the return wire and N turns of the toroidal core. The waveform representing the current can be converted to a voltage waveform by placing a resistor between the terminations of the toroidal core turns. This voltage waveform is substantially sinusoidal in nature and may require further modification. AC/DC converter circuits 408a-408d may be utilized to substantially convert the alternating current signal of the return current into a direct current signal. This eliminates any phase or frequency modulation that could lead to inaccuracies in measurement. This DC response is representative of the amount of RF current flowing through each return electrode 200a-200d. AC/DC converter circuit may be associated with each respective sensor 402a-402d.

Once the DC response of each return electrode 200a-200d is obtained, the signal may then be fed into a respective comparator 404a-404f. Each comparator 404a-404f receives two distinct DC inputs, each from a separate return electrode 200a-200d. One possible type of comparator is an instrumentation amplifier. Instrumentation amplifier receives a DC input from two different return electrode 200a-200d and calculates the current differential between the two. This difference is then multiplied by the gain of comparator or instrumentation amplifier 404a-404f in order to obtain a scaled representation of imbalances between any two of the return electrode 200a-200d. Ideally, the current differential would be negligible with each return electrode receiving the same amount of return current. However, if a substantial imbalance is present, a warning is provided via a suitable warning device (audible or visual) or safety control algorithms that are utilized to mitigate return electrode site burns.

Generator 120 may contain, *inter alia,* embedded software. This embedded software may be utilized to develop safety control algorithms or similar warning mechanisms. Using the information provided by comparator(s) 404a-404f, generator 120 may be able to modulate the amount of power delivered to each return electrode 200a-200d, thereby minimizing the chances of return electrode site burns. Moreover, this information may also be processed using a variety of suitable techniques, including but not limited to, neural networks or fuzzy logic algorithms.

A current sense transformer may be replaced with any current measuring device, such as a non-inductive sense resistor. Similarly, comparator or instrumentation amplifier could be replaced with a number of different devices including, but not limited to, differential amplifiers. Moreover, AC/DC converter circuit(s) 408a-408d may take on a number of suitable forms, such as a full-wave rectifier circuit.

To further limit the possibility of patient bums, an adhesive layer 500 may be disposed about the periphery of return electrode 200, as illustrated in FIGS. 2 and 3. The adhesive layer 500 may be conductive and may be made from materials that include, but are not limited to, a polyhesive adhesive; a Z axis adhesive; or a water-insoluble, hydrophilic, pressure-sensitive adhesive and is desirably made of a polyhesive adhesive. A function of the adhesive layer 500 is to ensure an optimal surface contact area between the return electrode 200 and the patient "P" thus limiting the possibility of a patient burn.

The return electrode(s) 200 may be entirely disposable, entirely re-usable, or a combination thereof. In one embodiment, the conductive elements 220 are re-usable, while the adhesive layer 500 is disposable. Other combinations of disposable/re-usable portions of the return electrode 200 are within the scope of the present disclosure.

As seen in FIG. 4, a multiplexer 260 may be employed to control switching of the plurality of conductive elements 220a-220c into and out of the circuit. For example, the multiplexer 260 may be configured to regulate the current in any fashion by switching "on" and "off" the individual conductive elements 220a-220c. While the multiplexer 260 is illustrated between the generator 120 and the connection device 300, other locations for the multiplexer 260 are within the scope of the present disclosure.

The present disclosure also includes a method for performing monopolar surgery. The method utilizes one or more return electrodes associated with a current detection system 400, as described above. The method also includes placing one or more return electrodes into contact with a patient, generating electrosurgical energy via an electrosurgical generator 120, supplying the electrosurgical energy to the patient via a surgical instrument 110, measuring the current density along the leading edge of each conductive element of each return electrode, detecting spikes and/or relatively large readings of the current in the leading edge of each conductive element and comparing said readings with predetermined levels, warning the user of a possible hazardous condition; and providing a means for substantially correcting the imbalances. The imbalances are corrected by removing from the circuit a conductive element exhibiting the current density level above said predetermined level.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of various embodiments. For example, the return electrode 200 may be at least partially coated with a positive temperature coefficient (PTC) material to help distribute the heat across the return electrode 200.

## Claims

1. A return electrode (200) for use in an electrosurgical system (100), the return electrode comprising:
a non-conductive pad (210) having a top surface and a bottom surface; and
a plurality of concentric, electrically isolated conductive elements (220 a-c) coupled to the top surface (212) of the non-conductive pad, concentrically outer conductive elements (220 a,b) surrounding concentrically inner conductive elements (220 b,c), wherein each conductive element defines a leading edge (222 a-c) to be located in relatively close proximity to a source (120) of electrosurgical energy in an electrosurgical system, wherein each conductive element is independently electrically connectable to the source of electrosurgical energy, **characterized in that**:
a current detection system (400) is connected to each conductive element (220 a-c) between the top surface (212) and the bottom surface (214) of the non-conductive pad (210).

2. The return electrode according to Claim 1, wherein the current detection system (400) includes at least one current sensor (402 a-f) coupled to a respective conductive element (220 a-c).

3. The return electrode according to Claim 2, wherein the at least one current sensor (402 a-f) is coupled to the leading edge (222 a-c) of the respective conductive element (220 a-c).

4. An electrosurgical system (100), comprising;
an electrosurgical generator (120); and
a return electrode (200) according to any preceding claim selectively connectable to the electrosurgical generator.

5. The electrosurgical system according to Claim 4, further comprising a connection device (300) for connecting the return electrode (200) to the electrosurgical generator (120).

6. The electrosurgical system according to Claim 4 or 5, further comprising a computer (180) electrically connected to each conductive element (220 a-c).

7. The electrosurgical system according to Claim 6, wherein the computer (180) is configured to independently switch each conductive element (220 a-c) into and out-of an electrical circuit.

8. The electrosurgical system according to Claim 6 or 7, wherein the computer (180) is configured to switch a conductive element (220 a-c) out-of the electrical circuit when a current density of the respective leading edge (222 a-c) of the conductive element (220 a-c) reaches a predetermined threshold level.

9. The electrosurgical system according to Claim 6, 7 or 8, wherein the computer (180) is configured to sequentially independently switch each conductive element (220 a-c) into and out-of the or an electrical circuit.

## Patentansprüche

1. Rückführelektrode (200) zur Verwendung in einem elektrochirurgischen System (100), wobei die Rückführelektrode umfasst:
ein nichtleitendes Feld (210) mit einer oberen Oberfläche und einer unteren Oberfläche; und
eine Mehrzahl konzentrischer, elektrisch isolierter Leitungselemente (220a-c), die an die obere Oberfläche (212) des nichtleitenden Feldes gekoppelt sind, konzentrische äußere Leitungselemente (220a, b), die konzentrische innere Leitungselemente (220b, c) umgeben, wobei jedes Leitungselement eine vordere Kante (222a-c) so definiert, dass sie in relativ naher Nähe einer Quelle (120) elektrochirurgischer Energie in einem elektrochirurgischen System angeordnet ist, wobei jedes Leitungselement unabhängig elektrisch mit der Quelle elektrochirurgischer Energie verbunden werden kann, **dadurch gekennzeichnet, dass**:
ein Stromdetektionssystem (400) mit jedem Leitungselement (220a-c) zwischen der oberen Oberfläche (212) und der unteren Oberfläche (214) des nichtleitenden Feldes (210) verbunden ist.

2. Rückführelektrode nach Anspruch 1, wobei das Stromdetektionssystem (400) zumindest einen Stromsensor (402a-f) enthält, der an ein entsprechendes Leitungselement (220a-c) gekoppelt ist.

3. Rückführelektrode nach Anspruch 2, wobei der zumindest eine Stromsensor (402a-f) an die vordere Kante (222a-c) des jeweiligen Leitungselements (220a-c) gekoppelt ist.

4. Elektrochirurgisches System (100), umfassend:
einen elektrochirurgischen Generator (120); und
eine Rückführelektrode (200) nach einem vorhergehenden Anspruch, die wahlweise mit dem elektrochirurgischen Generator verbunden werden kann.

5. Elektrochirurgisches System nach Anspruch 4, ferner umfassend eine Verbindungsvorrichtung (300) zum Verbinden der Rückführelektrode (200) mit dem elektrochirurgischen Generator (120).

6. Elektrochirurgisches System nach Anspruch 4 oder 5, ferner umfassend einen Computer (180), der elektrisch mit jedem Leitungselement (220a-c) verbunden ist.

7. Elektrochirurgisches System nach Anspruch 6, wobei der Computer (180) dazu ausgestaltet ist, unabhängig jedes Leitungselement (220a-c) in und aus einem elektrischen Schaltkreis zu schalten.

8. Elektrochirurgisches System nach Anspruch 6 oder 7, wobei der Computer (180) dazu ausgestaltet ist, ein Leitungselement (220a-c) aus dem elektrischen Schaltkreis zu schalten, wenn eine Stromdichte der entsprechenden vorderen Kante (222a-c) des Leitungselements (220a-c) einen vorbestimmten Schwellenwert erreicht.

9. Elektrochirurgisches System nach Anspruch 6, 7 oder 8, wobei der Computer (180) dazu ausgestaltet ist, nacheinander unabhängig jedes Leitungselement (220a-c) in und aus dem oder einem elektrischen Schaltkreis zu schalten.

## Revendications

1. Electrode de retour (200) pour utilisation dans un système électrochirurgical (100), l'électrode de retour comprenant:
un coussinet non conducteur (210) ayant une surface supérieure et une surface inférieure; et
plusieurs éléments conducteurs concentriques, électriquement isolés (220a-c) couplés à la surface supérieure (212) du coussinet non conducteur, des éléments conducteurs concentriquement extérieurs (220a, b) entourant des éléments conducteurs concentriquement intérieurs (220b, c), où chaque élément conducteur définit un bord avant (222a-c) à situer à proximité relativement étroite d'une source (120) d'énergie électrochirurgicale dans un système électrochirurgical, où chaque élément conducteur peut être connecté indépendamment électriquement à la source d'énergie électrochirurgicale, **caractérisée en ce que**:
un système de détection de courant (400) est relié à chaque élément conducteur (220a-c) entre la surface supérieure (212) et la surface inférieure (214) du coussinet non conducteur (210).

2. Electrode de retour selon la revendication 1, où le système de détection de courant (400) comporte au moins un capteur de courant (402a-f) couplé à un élément conducteur respectif (220a-c).

3. Electrode de retour selon la revendication 2, où le au moins un capteur de courant (402a-f) est couplé au bord avant (220a-c) de l'élément conducteur respectif (220a-c).

4. Système électrochirurgical (100), comprenant:
un générateur électrochirurgical (120); et
une électrode de retour (200) selon l'une quelconque des revendications précédentes, pouvant être connectée sélectivement au générateur électrochirurgical.

5. Système électrochirurgical selon la revendication 4, comprenant en outre un dispositif de connection (300) pour connecter l'électrode de retour (200) au générateur électrochirurgical (120).

6. Système électrochirurgical selon la revendication 4 ou 5, comprenant en outre un ordinateur (180) connecté électriquement à chaque élément conducteur (220a-c).

7. Système électrochirurgical selon la revendication 6, dans lequel l'ordinateur (180) est configuré pour commuter indépendamment chaque élément conducteur (220a-c) dans un circuit électrique et hors de celui-ci.

8. Système électrochirurgical selon la revendication 6 ou 7, où l'ordinateur (180) est configuré pour commuter un élément conducteur (220a-c) hors du circuit électrique lorsqu'une densité de courant du bord avant respectif (222a-c) de l'élément conducteur (220a-c) atteint un niveau de seuil prédéterminé.

9. Système électrochirurgical selon la revendication 6, 7 ou 8, où l'ordinateur (180) est configuré pour commuter séquentiellement indépendamment chaque élément conducteur (220a-c) dans et hors du ou d'un circuit électrique.
